# EUROPEAN PATENT SPECIFICATION

(11) **EP 2 313 114 B1**
(45) Date of publication and mention of the grant of the patent: **23.03.2022**
(21) Application number: 08775027.9
(22) Date of filing: 11.07.2008
(51) Int. Cl.: A61K 9/00, A61K 9/16, A61K 31/7036, A61P 11/00

(54) **A DRUG POWDER FOR INHALATION ADMINISTRATION AND A PROCESS THEREOF**
ARZNEIMITTELPULVER ZUR INHALATION UND VERFAHREN DAFÜR
POUDRE MÉDICAMENTEUSE DESTINÉE À UNE ADMINISTRATION PAR INHALATION ET PROCÉDÉ DE PRÉPARATION

(43) Date of publication of application: 27.04.2011
(73) Proprietor: Università degli Studi di Parma, 43100 Parma (IT)
(72) Inventor: BUTTINI, Francesca, I-43100 Parma (IT); COLOMBO, Paolo, I-43100 Parma (IT); PARLATI, Chiara, I-73100 Lecce (IT)
(74) Representative: Coppo, Alessandro
(86) International application number: PCT/EP2008/059110
(87) International publication number: WO 2010/003465

(56) References cited:
- WO-A-03/035051
- WO-A1-2007/068443
- WO-A2-2008/043825
- WO-A2-2008/053253
- US-A1- 2004 184 995
- VAN ZANDWIJK, N. ET AL: "Aerosol application of interferon-alpha in the treatment of bronchioloalveolar carcinoma." EUR. J. CANCER, vol. 26, no. 6, 1990, pages 738-740, XP002566544

## Description

### FIELD OF THE INVENTION

The present invention concerns a powder comprising an aminoglycoside antibiotic substance at least partially coated with a fatty acid or its salt and its use for inhalation for use

in the treatment of diseases derived from bacterial infections.

### STATE OF THE ART

Delivery of the poorly oral absorbed antibiotics such as aminoglicosydes to the airway by the aerosol route has been tested in cystic fibrosis patients. Much of this work has been done toward treatment of chronic lung infections with the administration of solutions by nebulization.

Cystic fibrosis (CF) is a common potentially fatal inherited disease affecting more than 60,000 people worldwide. It is associated with an wrongness in the transport of chloride ions across the epithelial membranes of exocrine glands, which causes a decreased water content of their secretions (Moskowitz, S.M., et al., 2005, Cystic fibrosis lung disease: genetic influences, microbial interactions, and radiological assessment. Pediatr. Radiol. 35, 739-757). Morphological changes of dilatation and hypertrophy of the bronchial glands are followed by mucous plugging. This viscid mucus in the airways allows bacterial colonization, with consequent infection of the respiratory tract, contributing to ongoing tissue damage. *Haemophilus influenzae* and *Staphylococcus aureus* are the first pathogens, which colonize the airway in childhood. As the lung disease progresses, colonization by *Pseudomonas aeruginosa* will follow. After a period of intermittent colonization with *P.aeruginosa* the colonization becomes chronic in most CF patients, and virtually impossible to be eradicated. CF patients are usually treated with two parenteral antipseudomonal antibiotics, one of which is typically an aminoglycoside. These antibiotics oppose the CF progression by reducing pulmonary deterioration and improving lung function.

Aminoglycosides are a group of antibiotics that includes at least eight drugs: amikacin, gentamicin, kanamycin, neomycin, netilmicin, paromomycin, streptomycin and tobramycin. They have the same basic chemical structure, i.e. aminocyclitol ring linked to aminosugars in their structure. Their bactericidal activity is attributed to the irreversible binding to the ribosomes, although their interaction with other cellular structures and metabolic processes has also been considered. They have a broad antimicrobial spectrum. They are active against aerobic and facultative aerobic Gram-negative bacilli and some Gram-positive bacteria of which staphylococci.

Intravenous administered aminoglycosides penetrate poorly into the endobronchial space, reaching mean peak sputum concentrations that are only 12 to 20% of the peak serum concentrations. However, as the components of CF sputum bind to aminoglycosides, sputum concentrations must exceed 10-fold to 25-fold the minimum inhibitory concentration to be bactericidal (Mendelman PM, Smith AL, Levy J, et al. Aminoglycoside penetration, inactivation, and efficacy in cystic fibrosis sputum. Am Rev Respir Dis 1985; 132:761-765). Thus, to achieve adequate drug concentrations at the site of infection, it is necessary to use large doses. A disadvantage of the aminoglycosides is their association with nephrotoxicity and ototoxicity, both of which are associated with elevated trough plasma levels and sustained elevated peak levels.

These problems can be overcome with the use of aerosolized aminoglycosides, which are suitable for inhalation because they remain bioactive when aerosolized and are poorly absorbed across epithelial surfaces. Thus, high concentrations in bronchial secretions can theoretically be achieved with minimal risk for systemic toxicity.

The goal of therapy with inhaled antibiotics in CF is to reach the entire bronchial tree from the upper tract, which may contain reservoirs of Pseudomonaes aeruginosa from where the lower airways can be infected, to the small peripheral airways where disease progresses. By taking into consideration the fact that the respiratory airways comprise nearly 95% of the total lung surface area, targeting of antibiotics in CF therapy should aim primarily at the peripheral small airways to reach sufficiently high drug concentrations throughout the respiratory system. On the other hand, aerosols are subject to impaction and gravitational sedimentation problems. Impaction occurs mainly with larger particles whenever the transportation is fast and changes direction, or is turbulent. Impaction, therefore, will take place in the upper airways, i.e. mouth, pharynx and larynx and large airways of the lung down to 2 µm in diameter. Sedimentation is a time dependent process, in which small aerosol particles settle in the airways under the influence of gravity. This takes place in the small airways and alveoli.

The most appropriate measure of aerosol particle size is the aerodynamic diameter, because it relates to particle dynamic behaviour and depends on the main mechanisms of aerosol deposition in the respiratory tract. This key physical parameter predicts the site of aerosol deposition within the lungs for micronized inhaled particles. It is the aerodynamic diameter of aerosol particles that defines the site of deposition of aerosol drugs: particles larger than 10 µm are deposited in the upper respiratory tract and have little therapeutic effect. Small particles with an aerodynamic diameter smaller than 5 µm pass trough the upper airways and deposit into the lower airways under gravity influence and Brownian motion. In contrast, particles smaller than 0.5 µm may not deposit at all, since they settle very slowly and can be dragged by the air-flow and finally, exhaled. Aerosols are usually hetero-disperse and their behaviour is described by the mass median aerodynamic diameter (MMAD). Half of the aerosol mass is contained in particles smaller and the other half of the aerosol mass is contained in particles larger than the MMAD.

There are five current classes of inhalation devices that can deliver drugs to the respiratory tract: dry powder inhalers, metered dose inhalers, metered dose nebulizers, ultrasonic nebulizers, and jet nebulizers. In their current form, metered dose inhalers are impractical to deliver the mass of aminoglycoside substances required for activity. Metered dose nebulizers are devices that produce aerosols by forcing a solution through a sieve at high pressure and have not been clinically tested for antibiotic delivery. To adequately treat the pulmonary lesion in CF, where involvement begins in the smaller bronchioles and extends towards the bronchi, the nebulizer should produce particles in the range 1-5 µm. However, there is a wide variation between different nebulizers in the mean size and range of sizes of particles generated. Newman et al. have evaluated several commercially available jet nebulizers for use with gentamicin solution (Newman SP, Pellow PGD, Clay MM, Clarke SW. Evaluation of jet nebulisers for use with gentamicin solution. Thorax1985; 40: 671-676. 1982; 4: 626-630). Aerosol output, droplet size and nebulization time were assessed. They found a ten-fold difference between the most and the least efficient delivery device. Efficiency increased with airflow rate and volume to be nebulized.

To date, the only inhalable aminoglycosidic antibiotic product available on the market is Tobi^{®}, a preservative-free tobramycin solution for nebulization that has been approved by the United States Food and Drug Administration (D. E. Geller, W. H. Pitlick, P. A. Nardella, W. G. Tracewell, B. W Ramsey. Pharmacokinetics and bioavailability of aerosolized tobramycin in cystic fibrosis. Chest (2002) 122 (1) pp. 219-26). Jet and the ultrasonic nebulizers are frequently used to delivery tobramycin solution to the lungs. The need for a compressor unit or pressurized air for the jet type and electricity for the ultrasonic nebulizer immobilizes the patient. Regular cleaning and disinfection of the nebuliser is required in order to prevent contamination with microorganisms and subsequent colonization of the patient's oropharynx. An even greater drawback of nebulisers is their low efficiency. For most nebulizer systems, it is estimated that approximately 10% of the mass of drug initially nebulized is deposited in the lungs and the remaining 90% either remains in the nebulizer, is impacted on the oropharynx and swallowed, or is dispersed into the atmosphere (Touw DJ, Brimicombe RW, Hodson ME, et al. Inhalation of antibiotics in cystic fibrosis. Eur Respir J 1995; 8:1594 -1604).

Dry powder inhalation may provide an alternative for drug nebulization. In a few studies, the use of micronized antibiotics has been reported. Goldman et al. have shown that inhaled micronised gentamicin powder produces gentamicin concentrations in bronchoalveolar lavage fluid similar to those produced by gentamicin solution nebulised (J.M. Goldman, S.M. Bayston, S. O'Connor, R.E. Meigh, Inhaled micronized gentamicin powder: a new delivery system, Thorax 45 (1990) 939-940). Inhaling a dry powder preparation is quick and convenient and may be particularly suitable for patients with cystic fibrosis and bronchiectasis who wish to maintain an independent life style.

However, micronized powders show poor flowability and extreme agglomeration and adhesion tendency. They have to be processed into a suitable free flowing formulation for accurate dose reproducibility, especially in the low dose-range, used for inhalation.

Recent particle-engineering technologies, such as the PulmoSphere process (Inhale Therapeutic Systems; San Carlos, CA), have led to easily dispersible powders with improved aerodynamic properties. A pulmonary delivery of an engineered tobramycin powder aerosol (tobramycin PulmoSphere formulation, *PStob*; Inhale Therapuetic Systems) from a simple, portable DPI (Dry Powder Inhaler,) has been described by Newhouse et al (Newhouse et al, 2003, Newhouse MT, Hirst PH, Duddu SP, Walter YH, Tarara TE, Clark AR, Weers JG: Inhalation of a dry powder tobramycin PulmoSphere formulation in healthy volunteers. Chest 2003;124:360-366). This study has demonstrated that pulmonary delivery of tobramycin sulphate from a passive DPI is feasible for *PStob* due to the high drug loading (90% weight/weight tobramycin sulfate), and nine-fold improved pulmonary deposition compared to the nebulizer over a fifteen-min administration period. A comparable dose can be delivered to the lung with *PStob* powder as with the nebulized product, but in approximately one-tenth the time and with considerably less effort. This has the potential to improve patient acceptance and adherence. The relatively large mass of powder administered over 15 min (150 mg, 52 mg deposited in the lung, and 65 mg deposited in the oropharynx) was well tolerated.

Pilcer et al. (Pilcer G., Sebti T., Amighi K: Formulation and characterization of lipid-coated tobramycin particles for dry powder inhalation. Pharm Res (2006) vol. 23 (5) pp. 931-40) demonstrated that the 15 minutes nebulization of 300 mg Tobi^{®} would be advantageously replaced with the inhalation of four 15-mg capsules or two 30-mg capsules of lipid-coated tobramycin. They used phospholipids and cholesterol coated tobramycin particles to improve drug targeting to the lung. The lipid coating modified the properties of micronized tobramycin particles, enabling lung deposition. Lipids used were phospholipids and cholesterol, two physiologically well-tolerated components used in concentration of 5% on tobramycin. The study demonstrated that the use of phospholipid compositions, based on mixtures of cholesterol and phospholipids, improved delivery of tobramycin to the pulmonary tract. Particles prepared were small and presented very high FPF (Fine Particle Fraction) results. Good flowability was observed, making the powders ideally suitable for use in carrier-free dry powder inhalers.

The authors therefore, selected two lipid formulations on the basis of their aerodynamic behaviour and fine particle dose (FPD) values and compared to Tobi^{®} (nebulizer solution) (Chiron Corporation; Seattle, WA) in a combined in vivo scintigraphic and pharmacokinetic evaluation of tobramycin after inhalation of a single oral dose in nine CF patients. The results of the study have demonstrated that Tobramycin DPI formulations containing high drug concentrations allow high lung deposition in patients. The inhalation of one capsule of 40 mg or two capsules of 20 mg of lipid-coated micronised tobramycin or two capsules of 30 mg of micronized tobramycin could be an advantageous substitute for the administration of Tobi^{®}.

For the inhalation use in dry powder inhalers, therefore it is felt the need of having drug substance powders transformed with appropriate techniques in respirable particle collections. Size, shape and density allow the particle to deposit in the appropriate area of the lung. In particular the relevant point is to have respirable particle containing the lowest adjuvant amount in order to avoid to charge the lung with non active potentially harmless substances. Therefore, the major problem for the administration by inhalation of some powder of aminoglycoside drugs is to transform its micromeritics characteristics in order to adapt it to be used in dry powders inhalers, avoiding the excessive use of adjuvant. Additional problems, other than they involved in aerodynamic properties, are the reduction of cohesiveness of small powder and their sensitivity to humidity of the environment or hygroscopic behaviour that opposes significantly the capability of the powder to be aerosolized or simply metered or also the preservation during the storage.

Aminoglycoside for lung administration present problems in manufacturing the micronized powders that have to be respirable, stable, highly drug concentrated and flowable. A frequently used micronization process for making inhalation powders is the spray drying procedure in which droplets of product dispersion or solution are dried in a flux of hot air.

The literature solutions of the problems of inhalation powders of tobramycin manufactured by spray drying consisted in spray drying dispersions of tobramycin micronized particles or emulsions of drug water solution in organic solvents.

Never the spray drying of tobramycin base water solution was performed.

The present inventors attempted to spray-dry tobramycin water solutions without excipients but we obtained powders too highly hygroscopic, deliquescent and non-stable at normal storage conditions. In these cases the respirability was lower than 30% but, more importantly, the powder could not be aerosolized due the high particle cohesivity and low flowability.

The use of lipid substances as adjuvant in tentative to reduce the water uptake and improve flowability was used by Pilcer G., but the described procedure required the use of previously micronized tobramycin powders. Since lipid substances are not soluble in water, the procedure for drug particle coating required organic solvents for homogeneously combination of the adjuvant with the drug. Thus, the authors spray dried a suspension of tobramycin previously micronized particles in organic solvents containing the dissolved lipids . Apart the problem of the use of organic solvents for lipid dissolution, tobramycin used in this procedure requires to be micronized in advance by mechanical treatment before to be spray-dried. In addition the tobramycin inhalation powders contained 5% w/w of phospholipids and cholesterol and the large amount of these substances introduce concerns on physico-chemical stability.

Tobramycin powder for inhalation was also manufactured using lipid-based PulmoSphere technology, producing highly dispersible porous particles. In this case, tobramycin particles have been made using an emulsion based tobramicyn sulphate solution containing a volatile blowing agent (perfluoroctylbromide) which aids in the creation of ultralow density sponge like particles. The lipid was phosphatidylcholine at high concentration in ratio to tobramycin used as emulsifier and the drug substance was the sulphate salt.

WO 2008053253 discloses the use of dry powder formulations which are prepared to achieve a high delivered dose of the active agent, wherein a force control agent is present on the surface of the active agent particles.

### SUMMARY

The above stated problems and the drawbacks of the prior art systems are overcome by a drug powder for inhalation comprising particles in which the drug content is equal to or higher than 90% w/w, wherein said drug particles are at least partially coated with an amount of an adjuvant lower than or equal to 2.0% w/w with respect to the weight of the drug, said adjuvant consisting of one or a mixture of C₁₂-C₁₈ saturated fatty acid or their salts, wherein said drug powder is obtained by
a) preparing a clear solution of the drug particles by mixing a water solution of the drug particles with an alcoholic solution of the adjuvant, wherein the adjuvant which consists of one or a mixture of C₁₂-C₁₈ saturated fatty acid or their salts is colloidally dispersed in the solution; and
b) spray drying the clear solution of step a) using an inlet temperature between 120-135 °C, feed rate between 3.0 and 6.0 ml/min, air flow rate 600 L/h, nozzle between 0.5 and 1 mm, and aspirator 100%, wherein
   the temperature of the clear solution in step a) and step b) is higher than or equal to 28°C to colloidally disperse the adjuvant in the solution, and wherein said drug powder is an aminoglycoside antibiotic comprising particles of antibiotic which have a solubility in water of at least 3.0% w/v.

In the present disclosure:
- when "drug" is used it is intended to refer to aminoglycoside antibiotic substances that have a solubility in water of at least 3.0% w/v;
- when "at least partially coated" is used for a drug particle, it is intended that the drug particle has at least more than 20% of the particle surface coated, preferably more than 50%; more preferably the coating is in the form of spots.

In another aspect a process is described for preparing the drug powder, which allows the transformation of the micromeritic properties i.e., particle size, shape and density characteristics of aminoglycoside antibiotics substances, in order to make the powder respirable, stable, highly concentrated and flowable, i.e., suitable for inhalation as dry powder inhalers.

The product as disclosed herein comprises lipids such as saturated fatty acids having different chain length or their salts. These lipids are not miscible with the water solution and an intimate mixing capable to distribute the lipid on the drug particle surface formed during drop drying is not feasible. A process is herein described, which provides for the preparation of water dispersion to be sprayed, in which the lipid adjuvant was colloidal dispersed in the drug water solution. The procedure invented was capable to provide a solution of drug and adjuvant, which remained limpid without separation of the components for the time required by the spray drying of solution.

Therefore, the process for the preparation of the drug powder comprises the following steps:
a) preparing a clear solution of drug particles by mixing a water solution of the drug particles with an alcoholic solution of the adjuvant, wherein an adjuvant which consists of one or a mixture of C₁₂-C₁₈ saturated fatty acid or their salts is colloidally dispersed in the solution; and
b) spray drying the clear solution of step a) using an inlet temperature between 120-135 °C, feed rate between 3.0 and 6.0 ml/min, air flow rate 600 L/h, nozzle between 0.5 and 1 mm, and aspirator 100%, wherein

the temperature of the clear solution in step a) and step b) is higher than or equal to 28°C to colloidally disperse the adjuvant in the solution, and wherein said drug powder is an aminoglycoside antibiotic comprising particles of antibiotic which have a solubility in water of at least 3.0% w/v.

In the present process the solution is clear, because the adjuvant is colloidally dispersed in the solution. In order to colloidally disperse the adjuvant the temperature of the solution in step a) and in step b) would be selected on basis of the kind of C₁₂-C₁₈ saturated fatty acid. Such a temperature is higher than or equal to 28°C, more preferably higher than or equal to 30°C, still more preferably of about 30°C.

This solution was obtained by an appropriate mixture of water solution of drug with an alcoholic solution of the adjuvant. In this way, during the drying of the droplet produced in the spray drying apparatus, due to the solvent evaporation, the adjuvant precipitates at the water/air interface of the drop forming drug particle. The procedure adopted and the ingredients used determined the deposition of the saturated fatty acid, preferably as spots, on the surface of the dried drug particle.

The result of the process is the attainment of a drug particle, intended for the treatment of chronic bacterial broncho-pulmonary infection and exhibiting high respirability, stability, elevated content of active principle, without affecting the drug dissolution and flowability

### DESCRIPTION OF THE FIGURE

Certain embodiments will be now described in the below detailed description with reference to the following figures, wherein:
- Figure 1 shows a typical spray dried particle of tobramycin obtainable by the present Example 1 and Example 2, respectively; and
- Figure 2 illustrates the presence of the adjuvant on the drug particle surface obtained in the present Example 3, in dependence on the amount of adjuvant used in the preparation of the solution.

### DETAILED DESCRIPTION OF THE INVENTION

The invention relates to a drug powder as defined in claim 1.

The powder of the invention comprises particles of antibiotic, which is an aminoglycoside antibiotic and have a solubility in water of at least 3.0% w/v. Preferably, the aminoglycosidic antibiotic substance is selected from the group consisting of amikacin, gentamicin, kanamycin, neomycin, netilmicin, paromomycin, streptomycin and tobramycin, still more preferably it is tobramycin or amikacin.

The drug particle of the invention is at least partially coated with an adjuvant in an amount lower than or equal to 2.0% w/w with respect to the weight of the drug, which consists of one or a mixture of C₁₂-C₁₈ saturated fatty acid or their salts. The adjuvant will be preferably selected from the group consisting of lauric acid, myristic acid, palmitic acid, stearic acid, their mixtures or their salts. More preferably the adjuvant of the invention is stearic acid or a salt thereof.

Lauric acid has 12 carbons and is one of the three most widely distributed saturated fatty acids found in nature (14:0, 16:0, and 18:0). Myristic acid has 14 carbons. Palmitic acid has 16 carbons and it is the commonest saturated fatty acids in plant and animal lipids. Finally, stearic acid has 18 carbons and it is the highest molecular weight saturated fatty acid occurring abundantly in fats and oils.

The adjuvant can be also in the form of a salt of a C₁₂-C₁₈ saturated fatty acid. Preferably the salt is made with alkaline or terrous-alkaline metals, more preferably alkaline metals and, still more preferably, it is a salt of sodium.

The drug particle of the invention is at least partially coated with an amount below than or equal to 2.0% w/w of an adjuvant with respect to the weight of the drug. The adjuvant amount is preferably in the range from 0.25 to 2.0 w/w, more preferably in the range from 0.5 to 1.5.

The present invention relates also to a process for the preparation of the drug powder which comprises the following steps:
a) preparing a clear solution of drug particles by mixing a water solution of the drug particles with an alcoholic solution of the adjuvant, wherein an adjuvant which consists of one or a mixture of C₁₂-C₁₈ saturated fatty acid or their salts is colloidally dispersed in the solution; and
b) spray drying the clear solution of step a) using an inlet temperature between 120-135 °C, feed rate between 3.0 and 6.0 ml/min, air flow rate 600 L/h, nozzle between 0.5 and 1 mm, and aspirator 100%, wherein
   the temperature of the clear solution in step a) and step b) is higher than or equal to 28°C to colloidally disperse the adjuvant in the solution, and wherein
   said drug powder is an aminoglycoside antibiotic comprising particles of antibiotic which have a solubility in water of at least 3.0% w/v.

Without being bound to any theory, the inventors have found out that by preparing a clear solution of the drug and the adjuvant, a drug powder with high respirability and optimal physical and chemical features could be obtained by spraying drying such a solution.

In the present process the solution is clear and the adjuvant is colloidally dispersed in the solution. Such a temperature is higher than or equal to 28°C, more preferably higher than or equal to 30°C, still more preferably of about 30°C. According to a preferred embodiment, the process provides for preparing a solution of drug and separately a solution of adjuvant in order to obtain the clear mixture of step a).

Preferably, the drug is dissolved in water, more preferably in an amount in the range from 7 to 30 mg/ml and the adjuvant is dissolved in an alcohol, more preferably in an amount in the range from 0.15 to 0.70 mg/ml. The alcohol used for the dissolution of the adjuvant is preferably selected from the group consisting of ethanol, methanol, propanol or isopropanol.

In the preferred embodiment, the solutions of adjuvant and of drug are mixed in a ratio in the range from 2:8 to 5:5 more preferably 3:7.

In the most preferred embodiment the ethanolic solution of stearic acid is mixed with water solution of tobramycin in a ratio of 3:7.

The limpid solution of the invention in case of tobramycin preferably has pH values between 10.0-10.7.

The clear solution of step a) contains a concentration of solids which is below than or equal to 2% w/v, preferably in the range from 1% to 2% w/v.

In case of the preferred embodiment, wherein the drug is tobramycin and the adjuvant is sodium stereate, the concentration of solids of about 1% w/v was optimal in order to have the desired coating.

According to the present description, after the mixing of the two relevant solutions, a clear solution, wherein the adjuvant is colloidally dispersed is obtained. This solution is then dry-sprayed in step b) in order to obtain the drug powder. The obtained solution is preferably spray-dried in a Buchi 191 apparatus using an inlet temperature between 120-135 °C, preferably at about 125°C, feed rate between 3.0 and 6.0, preferably about 3.5 ml/min, air flow rate 600 L/h, nozzle between 0.5 and 1 mm, preferably 0.7 mm and aspirator 100%. According to the invention the solution to be dry-sprayed is limpid and kept limpid by maintaining its temperature higher than or equal to 28°C, more preferably higher than or equal to 30°C, still more preferably of about 30°C.

In this way, during the drying of the droplet produced in the spray drying apparatus, due to the solvent evaporation, the adjuvant precipitates at the water/air interface of the drop forming drug particle. The procedure adopted and the solvent used determined the deposition of the adjuvant on the surface of the dried drug particle.

The inventors therefore found out that the intimate mixing obtained was capable to concentrate the adjuvant at least partially on the surface of drug particle during drying. In the process according to the invention, a solution of drug and adjuvant was prepared, which remains limpid without separation of the components for the time needed to spray drying the solution. As described in the most preferred embodiment, the inventors found out that the two solutions could be spontaneously mixed in the water/alcohol ratio 70:30 with a gentle mixing, providing a limpid mixture at 30°C in which the adjuvant is colloidal distributed. The obtained powder useful for inhalation, preferably of tobramicyn or amikacin, is therefore characterized by a high content of drug substance. The present invention resolved the problems related to the difficulty of aerosolizing drug powder in which the particles do not possess a useful aerodynamic behaviour since they are too hygroscopic. The process according to the invention, which provides for spray drying a limpid solution of water-drug in the presence of very small amount of adjuvant, allowed to prepare drug powders with highly respirability, high drug content, not being hygroscopic, flowable and sufficiently resistant to the effect of uncontrolled environment exposure.

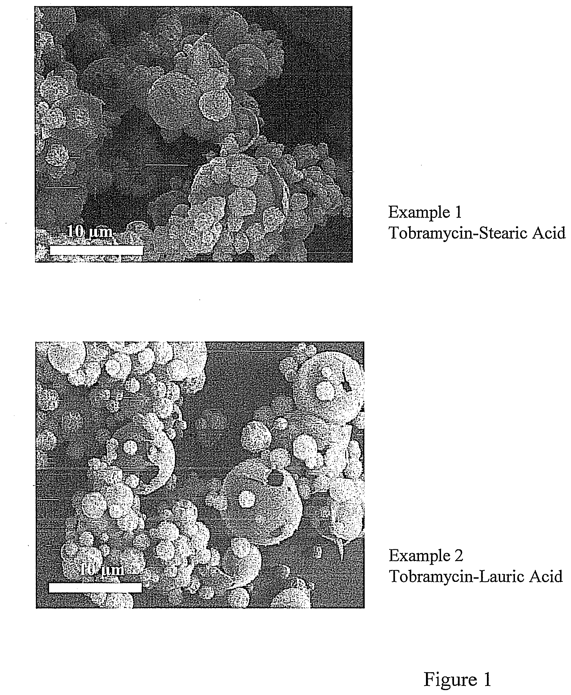

Due to the procedure of manufacturing, the pulmonary powders of tobramycin subject of the present invention are characterized by microparticles with corrugated surface as it will be evident from the annexed Figure 1. In case of tobramycin, the structure is completely different from the tobramycin microcrystal structure. These pulmonary powders of antibiotics show packing characteristics that allow them to be directly introduced in the reservoir of DPI delivery systems. In addition, this invention permits the aerosol administration of the substance in dry forms at high concentration, improving the storage stability and the dose deposited into the lungs.

According to the process herein disclosed, the transformation of the micromeritic properties i.e., particle size, shape and density, of aminoglycoside antibiotics substances is obtained in order to make them suitable for dry powder inhalation. The surprising technical feature consists in the preparation of solid particles by spray drying of water solution of the drug in which an adjuvant consisting of one or a mixture of C₁₂-C₁₈ saturated fatty acid or their salts is colloidally dispersed. The mixing procedure adopted allows the obtainment at a temperature higher than or equal to 28°C, more preferably higher than or equal to 30°C, still more preferably of about 30°C of a completely clear solution of the fatty acid lipid in water. During the drying, the colloidal structure of the solution promotes the formation of drug microparticles whose surface is covered by the adjuvant, in this way avoiding the need to micronize in advance the particles of the powder as described in the prior art. The amount of adjuvant present on the particle is very low (0.25-2.0 % w/w on tobramycin), definitely lower than prior art. For the powder of the invention, in spite of the low amount of adjuvant material (preferred 0.5-1.5% w/w on tobramycin), the sensitivity to humidity was significantly reduced and the aerosolization of the powder strongly improved. Therefore, according to the inventors, it is the adjuvant, colloidally dispersed in the antibiotic water solution that deposited on the antibiotic particles formed with the described procedure provides a protection from environment. In addition, the characteristics of size, shape and density of spray-dried particles make them favourable for lung deposition. As it will be described in the example 3, this deposition has been demonstrated using a scanning electron microscopy assisted by electron emission field probe.

Briefly, the process described herein was able to change the properties of the drug powder compared to the raw material, maintaining a high drug content (≥90%). In particular, the small amount of adjuvant was able to protect the powders from the humidity of the environment giving a non-hygroscopic behaviour to powders. In addition, tobramicyn particle obtained appeared corrugated in shape by the presence of the adjuvant, a feature that connected with the density and size, provided high respirability of the product. The new "nature" of the drug powder let to keep the product stable in the device for inhalation until its use. More relevant was the fact that the respirability of the powders having these properties was higher that the usual values described in literature. This respirability has been assessed using the Andersen Cascade Impactor or the Next Generation Impactor as described in the present United State Pharmacopoeia. These apparatuses are used to determine the fine particles of an aerosol cloud generated by preparations for inhalation and allow the measure of the mass of drug less than a particular aerodynamic particle size. The mass of drug having aerodynamic diameter lower than 5 µm is generally considered as "respirable", even though the optimal size for lung deposition is in the range 2 -5 micron. The process described in this patent provided the production of fine powders in which more than 90 % of particles have dimensions less than 7 microns as volume-diameter. On the other hand, the respirability study by Andersen Cascade Impactor and Next Generation Impactor of the powder of the invention was in all the cases higher than 50%.

Therefore, the optimal features of the drug powder of the invention is based on the finding that the peculiar corrugated microparticles obtained by spray-drying of a limpid water-alcohol solution of a drug and a fatty acid solution are micronized, free flowing and with high tapped density. The microparticles are essentially amorphous and characterized by a shape defined corrugated as shown in Figure 1 for tobramycin. This particle shape makes the powders not cohesive since the microparticles maintain their individuality and do not agglomerate. In addition, they show substantially no losses of activity if stored in normal conditions. These powders other than to be micronized, are non cohesive, quite free flowing and easy metered in the DPI. These physical properties, together with the favourable aerodynamic behaviour due to the size, shape and density of the particles, determine an unexpected and surprisingly high respirability.

Finally, the drug powder of the invention has drug content higher than or equal to 90%, since the residual moisture was lower than 8% w/w and the lipid content less than or equal to 2.0% w/w. They can be stored at normal humidity and temperature conditions in sealed container. The fact the final powder contains a very low percentage of the adjuvant makes the substance to be administered pretty pure. The administration here described can involve powdered drugs given as aerosol using a device capable to meter and aerosolize the particles. Furthermore, the powder of the present invention shows a respirable fraction of its aerosol higher than 40%, preferably > 48%.

The powders attainable with the procedure hereunder described possess the following characteristics that make them very suitable for the inhalation administration by a dry powder inhaler:
Yield of spray drying process (%): between 64% and 79%
Water content (%). between 5.4 and 9.0
Drug content (%): between 90 and 94
Aerodymanic size (MMAD): between 2.7µm and 4.6µm
Respirability (Fine Particle Fraction): between 41% and 53%
Packing: bulk density 0.14-0.26 g/ml; tapped density 0.23-0.40 g/ml
True density: between 1.16 g/ml and 1.60 g/ml
Carr Index: (measurement of the improvement in packing of a powder bed: values of about 20-30% indicate good flow) between 31.3% and 37.1%

The invention will be now described with reference to some examples, which have been provided for exemplificative purposes.

### EXAMPLES

### Example 1

This example describes the manufacture of a powder of tobramycin suitable for pulmonary administration. This example describes the preparation of the solution to be sprayed, in which stearic acid, as adjuvant, was colloidally dispersed in the tobramycin water solution in order to provide a limpid mixture of the components. The result of the process is the attainment of drug particle exhibiting stability and respirability without affecting the dissolution of the drug.

Ingredients and amounts for preparing 2 Litres of drug-adjuvant solution to spray drying, was as in Table 1.

**Table 1**

| COMPONENTS | Weigh of substances (g) |
|---|---|
| Tobramycin | 19.8 |
| Stearic Acid | 0.2 |
| Water | 1400 |
| Ethanol 95% | 490 |

The ratio of percentages between tobramycin and stearic acid was 99:1. Two solutions were prepared:
- a water solution containing the dissolved drug and
- an ethanolic solution containing the adjuvant.

The ratio between tobramycin-water and stearic acid-ethanol solution was 70:30. The stearic acid was dissolved in ethanol by heating the solution to 30°C and magnetically stirring at 200 rpm on a heated stirring plate for a 30 min in order to achieve a limpid transparent solution. A protection was employed to avoid the ethanol evaporation.

The tobramycin was dissolved in deionised water (conductivity 0.5 - 1.0 µS) at the concentration 14 mg/ml under magnetic stirring. After the heating of the solution of drug to 30°C, the stearic acid/ethanol solution was added at the rate of 100 ml /min stirring at 200 rpm for 15 min. The colloidal dispersion appears colourless, transparent and it is kept clear under stirring at a temperature of about 30°C during all the process of spray drying. The concentration of solids (drug + adjuvant) into the final solution was 1% w/v.

The resultant hydroalcoholic colloidal solution, in which the ratio between water and alcohol was 7:3, was spray dried on a Mini Spray Dryer B-191 (Buchi, Switzerland) using an inlet temperature of 125°C, material feed rate of 3 ml min-1, atomization flow of 100% and nozzle air flow of 600 ml min-1. The yield of the process to prepare tobramycin-stearic acid powder was 64.7% ± 3.4.

The attached Figure 1 shows the scanning electron microscopy picture of tobramycin/stearic acid microparticles. The tobramycin-stearic acid spray-dried powder showed a median volume diameter (dv 0.5) value of 3.65 ± 0.19. The Scanning Electron Microscopy morphological analysis of the powder indicated that the spray-dried tobramycin microparticles had a quite spherical shape with corrugated surface. The aerodynamic behavior was investigated using the Next Generation Impactor, determining the aerodynamic particle size distribution of the powder. The Fine Particle Fraction (FPF) (≤5 µm) value was 60% and the Median Mass Aerodynamic Diameter was around 3 µm. This powder showed a very good respirability, reached the deep stages of the Next Generation Impactor and the percentage recovered into the throat was low (7%).

### Example 2

This example describes the preparation of the solution to be sprayed in which the lauric acid was colloidally dispersed in the tobramycin water solution providing a limpid mixture of the components. Tobramycin-lauric acid microparticles were produced by spray-drying the drug with the lipophilic adjuvant. The ratio of percentages between tobramycin and lauric acid was 99:1. The concentration of solids (drug + lipophilic adjuvant) into the final solution was 1% w/v. A water solution of drug and an ethanolic solution of the lauric acid adjuvant were prepared. The ratio of percentages between tobramycin-water and lauric acid-ethanol solution was 70:30.

The composition for preparing 4 Liters of drug-adjuvant solution to spray drying, was as in Table 2.

**Table 2:**

| COMPONENTS | Weigh of substances (g) |
|---|---|
| Tobramycin | 39.6 |
| Lauric Acid | 0.4 |
| Water | 2800 |
| Ethanol | 980 |

Lauric acid was dissolved in ethanol by heating the solution to 30°C and stirring at 200 rpm on a heated stirring plate at for a further 30 min achieving a limpid transparent solution. Tobramycin was dissolved in deionised water (conductivity 0.5 - 1.0 µS) at the concentration 14 mg/ml. After the heating of the solution of drug to 30 °C, the lauric acid/ethanol solution was added at the rate of 100 ml /min stirring at 200 rpm for 15 min. The solution obtained at 30°C was clear.

The resultant hydroalcoholic colloidal solution was spray dried on a Mini Spray Dryer B-191 as in Example 1. The yield of the tobramycin-lauric acid dry powder preparation was 68.3% ± 2.6.

The tobramycin-lauric acid spray-dried powder showed a median volume diameter (dv 0.5) value of 3.03 ± 0.12. The Scanning Electron Microscopy morphological analysis of the powder indicated that the spray-dried tobramycin microparticles had a quite spherical shape with corrugated surface as shown in Figure 1. The aerodynamic behavior was investigated using the Next Generation Impactor, obtaining the aerodynamic particle size distribution of the powder. The Fine Particle Fraction (≤5 µm) value was 53% and the Median Mass Aerodynamic Diameter was around 3 µm. This powder showed a very good respirability, reached the deep stages of the Andersen Cascade Impactor and the percentage recovered into the throat was low (14%).

### Example 3

This example describes the production of a tobramycin-sodium stearate (99:1) powder for inhalation using a salt of fatty acid i.e., sodium stearate, added as adjuvant to lend stability and aerodynamic properties to tobramycin powder.

The composition for preparing 1 Litre of drug-adjuvant solution to spray drying, was as in Table 3:

**Table 3**

| COMPONENTS | Weigh of substances (g) |
|---|---|
| Tobramycin | 9.9 |
| Sodium Stearate | 0.1 |
| Water | 700 |
| Ethanol | 245 |

Tobramycin-sodium stearate microparticles were produced by spray-drying the drug-adjuvant solution. The ratio of percentages between tobramycin and sodium stearate acid was 99:1. The concentration of solids (drug + adjuvant) into the final solution was 1% w/v. The water solution of drug and the ethanolic solution of the lipophilic adjuvant at 30°C were mixed tobramycin-water and sodium stearate-ethanol solution ratio was 70:30.

The sodium stearate was dissolved in ethanol by heating the solution to 30°C and stirring at 200 rpm on a heated stirring plate at for a further 30 min in order to achieve a limpid transparent solution. The tobramycin was dissolved in deionised water (conductivity 0.5 - 1.0 µS) at the concentration 14 mg/ml and stirred for a further 10 min. After the heating of the solution of drug to 30 °C, the sodium stearate solution was added at the rate of 100 ml /min stirring at 200 rpm for 15 min. A clear solution was obtained.

The resultant hydroalcoholic colloidal solution was spray dried on a Mini Spray Dryer B-191 as in the example 1. The yield of the process to prepare a tobramycin-sodium stearate dry powder was 72.4% ± 1.9. Drug Content was 90.74%.

As it is known tobramycin is a highly hygroscopic powder, the crystals tend to absorb water from the environment to get liquefied. The powder prepared in this example contained an adjuvant which helped to protect the powder from the humidity and to decrease the hygroscopicity compared to the tobramycin raw material.

Scanning Electron Microscopy morphological analysis of the powder indicated that the spray-dried tobramycin microparticles had a quite spherical shape with corrugated surface. The Energy Dispersive Spectroscopy analysis of the sodium distribution on the surface of the microparticles indicted the maximum distribution of the adjuvant on the surface of the microparticles at the content 1 %. The water content in the tobramycin/sodium stearate powder determined by Karl Fisher method was 8.47 ± 0.53. The particle size distribution of the micronized tobramycin/sodium stearate dry powder was determined using laser diffraction (Mastersizer X, Malvern, UK). Particle size of tobramycin raw material and tobramycin spray-dried powder. are described in the following Table 4. The dv 0.1, dv 0.5, dv 0.9 represent the volume diameter of the particles at 10, 50 and 90% on the cumulative undersize curve.

**Table 4**

| | | | | |
|---|---|---|---|---|
| Polvere | dv 0.1 (µm) | dv 0.5 (µm) | dv 0.9 (µm) | Span |
| Tobramycin r.m. | 0.89 ± 0.02 | 4.01 ± 0.02 | 16.02 ± 0.51 | 1.87 ± 0.04 |
| Tobramycin SD | 1.11 ± 0.03 | 3.29 ± 0.44 | 8.06 ± 0.96 | 2.11 ± 0.09 |

The Andersen Cascade Impactor (ACI) fitted with a stainless steel 90-degree induction port was used according to the manufacturers instructions which included setting the flow rate at 60 L/min. During impact the aerosol was divided into seven size categories according to the particle aerodynamic diameter (USP 30). A Turbospin (Ph&T, MI, IT) device was in use in this study to aerosolize the powder. The respirable fraction was expressed in terms of fine particle fraction (FPF), which was deemed to be those particles smaller than 5 µm. The MMAD is defined as the median particle diameter of the formulation depositing within the ACI. The following Table 5 shows aerosolisation properties of the tobramycin/sodium stearate microparticles. As shown in Table 5, the prepared powder showed very good aerosolization properties.

**Table 5**

| | Aerodynamic Assessment | | | | | |
|---|---|---|---|---|---|---|
| | Label Dose (mg) | Emitted Dose (mg) | Recovery (%) | MMAD (µm) | FPD (mg) | FPF (%) |
| Tobramycin SD | 20 | 17.5 ± 0.16 | 87.2 ± 0.5 | 4.19 ± 0.29 | 7.51 ± 0.43 | 51.53 ± 3.16 |

The lipophilic adjuvant, in this case sodium stearate, distribution on the surface of the tobramycin microparticles was analyzed using SEM-EDS (Scanning Electron Microscopy-Energy Dispersive Spectroscopy, JSM 6000F JEOL, Japan). High resolution field emission microscope (0.6nm at 30kV), Windowless EDS microanalysis (to Boron).

Energy dispersive X-ray analysis, also known as EDS, EDX or EDAX, is a technique used to identify the elemental composition of a sample or small area of interest on the sample. During EDS, a sample is exposed to an electron beam inside a scanning electron microscope (SEM). These electrons collide with the electrons within the sample, causing some of them to be knocked out of their orbits. The vacated positions are filled by higher energy electrons, which emit x-rays in the process. By analyzing the emitted x-rays, the elemental composition of the sample can be determined. EDS is a powerful tool for microanalysis of elemental constituents.

These studies were conducted in order to try to identify any differences between the particles surface composition, in our case the sodium stearate presence. It would be difficult to evaluate the topography and composition of a particulate material by analysing a relatively small number of samples, however, these techniques may afford some information concerning the chemical composition of any particle surface features.

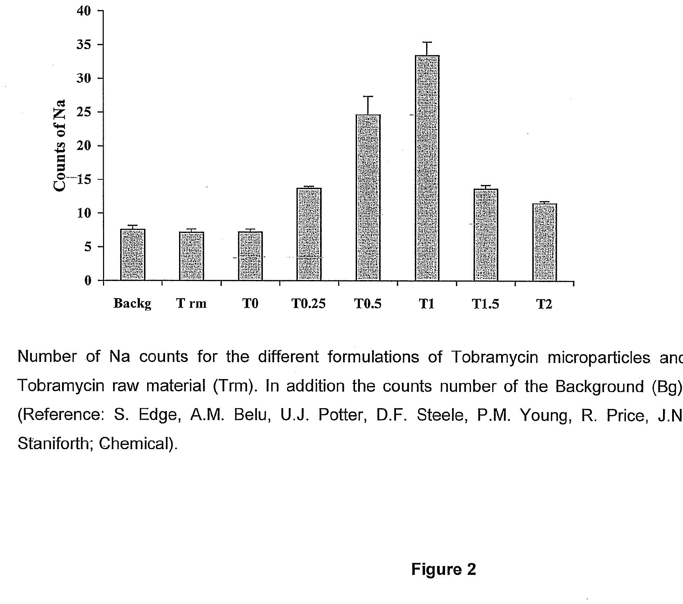

The EDS analysis indicated a distribution of adjuvant on the surface of the tobramycin microparticles. A linear relationship between sodium stearate concentration and sodium count was observed (R²= 0.97) up to a concentration of 1% as shown in Figure 2.

The content 1% was considered optimal since the increase of adjuvant from 0 to 1% determine the deposition on the drug particle of an amount of sodium stearate increasing linearly; further increase reduces the deposition on the surface of the microparticles of tobramycin. Indicating that the maximum amount of coverage was reached.

### Example 4

This example describes the production of an amikacin-sodium stearate (99.5:0.5) powder for inhalation using a salt of fatty acid i.e., sodium stearate, added as adjuvant to lend stability and aerodynamic properties to amikacin powder.

The colloidal dispersion preparation in which an ethanolic solution of sodium stearate and amikacin water solution was mixed using the process herein disclosed.

The composition for preparing 1 Liter of drug-lipophilic adjuvant solution to spray drying, was the following:

**Table 6**

| COMPONENTS | Weigh of substances (g) |
|---|---|
| Amikacin | 9.95 |
| Sodium Stearate | 0.05 |
| Water | 700 |
| Ethanol | 245 |

The sodium stearate was dissolved in ethanol at the concentration of 0.17 mg/ml by heating the solution to 30°C and stirring at 200 rpm on a heated stirring plate at for a further 30 min in order to achieve a limpid transparent solution. The amikacin was dissolved in deionised water (conductivity 0.5 - 1.0 µS). After the heating of the solution of drug to 30°C, the sodium stearate solution was added at the rate of 100 ml /min stirring at 200 rpm for 15 min. The water solution of drug and the ethanolic solution of the lipophilic adjuvant were mixed in ratio of 70:30. A limpid mixture of the components was obtained. The concentration of solids (drug + adjuvant) into the final solution was 1% w/v.

The hydroalcoholic colloidal solution was spray dried on a Mini Spray Dryer B-191 (in the same conditions of example 1). The yield of the process to prepare a tobramycin-sodium stearate dry powder was 79.4% ± 2.8.

The amikacin-sodium stearate spray-dried powder showed a median volume diameter (dv 0.5) value of 3.28 ± 0.19 smaller than 5 µm. The water content of the powder was 5.42% ± 0.64. The aerodynamic behavior was investigated using the Andersen Cascade Impactor, obtaining the aerodynamic particle size distribution of the powder. The Fine Particle Fraction (<5 µm) value was 41.7% ± 3.6 and the Median Mass Aerodynamic Diameter was around 4.6 µm. This powder showed a very good respirability, reached the deep stages of the Andersen Cascade Impactor and the percentage recovered into the throat was around 21%.

### Example 5

This example describes the production of tobramycin-sodium stearate (99:1) powder for inhalation using a salt of fatty acid i.e., sodium stearate in case the concentration of solids (drug + adjuvant) into the final solution was 2% w/v. This let the reduction of the volume and the time of the spray drying process.

The colloidal dispersion preparation in which an ethanolic solution of sodium stearate and tobramycin water solution was mixed using the herein described process.

Tobramycin-sodium stearate microparticles were produced by spray-drying the drug-adjuvant solution. The ratio of percentages between tobramycin and sodium stearate acid was 99:1. The water solution of drug and the ethanolic solution of the lipophilic adjuvant were mixed at 30°C. Tobramycin-water and stearate sodium-ethanol solution ratio was 70:30. The concentration of solids (drug + adjuvant) into the final solution was 2% w/v.

The composition for preparing 500 ml of drug-lipophilic adjuvant solution to spray drying, was the following:

**Table 7**

| COMPONENTS | Weigh of substances (g) |
|---|---|
| tobramycin | 9.9 |
| Sodium Stearate | 0.1 |
| Water | 350 |
| Ethanol | 123 |

The sodium stearate was dissolved in ethanol at the concentration of 0.6 mg/ml by heating the solution to 30°C and stirring at 200 rpm on a heated stirring plate at (Arex Velp Scientifica, MO, IT) for a further 30 min in order to achieve a limpid transparent solution. A protection was employed to avoid the ethanol evaporation.

The tobramycin was dissolved in deionised water (conductivity 0.5 - 1.0 µS) at the concentration 28.2 mg/ml and stirred for a further 10 min. After the heating of the solution of drug to 30 °C, the sodium stearate solution was added at the rate of 100 ml /min stirring at 200 rpm for 15 min.

A limpid mixture of the components was obtained.

The resultant hydroalcoholic colloidal solution was spray dried on a Mini Spray Dryer B-191 at the same conditions stated in Example 1.

The yield of the process to prepare a tobramycin-sodium stearate dry powder was 76.9% ± 2.1. The tobramycin-sodium stearate spray-dried powder showed a median volume diameter (dv 0.5) value of 2.59 ± 0.01. Water content in the tobramycin/sodium stearate powder was 9 %. The true density (g/ml) and Carr Index % of the powder were respectively 1.60 g/ml and 35.5%.

The aerodynamic behaviour was investigated using the Next Generation Impactor, obtaining the aerodynamic particle size distribution of the powder. The Fine Particle Fraction (<5 µm) value was 48.2% ± 3.9 and the Median Mass Aerodynamic Diameter was around 2.7 µm.

As shown in the above examples the preparation of solid particles by spray drying of water solution of the raw material in which a fatty acid in very low amount is colloidally dispersed, gives rise to an high content of drug, always more or equal to 90%. The present invention has solved the problems related to the difficulty of aerosolizing an antibiotic or chemotherapic powder in which the particles do not possess a useful aerodynamic behaviour. In addition, this invention permits the administration of the substance in dry forms improving the storage stability and the dose deposited into the lungs. Moreover, since the dose administered is often very high, the concentration of active substance allows to reduce the number of administrations and the aerosol density.

Due to the procedure of manufacturing, the pulmonary powders are characterized by structure, size and shape of the constituent microparticles with corrugated surface, completely different from the microcrystal structure. These pulmonary powders show packing characteristics that allow them to be directly introduced in the reservoir of DPI delivery systems.

## Claims

1. A drug powder for inhalation comprising particles in which the drug content is equal to or higher than 90% w/w, wherein said drug particles are at least partially coated with an amount of an adjuvant lower than or equal to 2.0% w/w with respect to the weight of the drug, said adjuvant consisting of one or a mixture of C₁₂-C₁₈ saturated fatty acid or their salts, wherein said drug powder is obtained by
a) preparing a clear solution of the drug particles by mixing a water solution of the drug particles with an alcoholic solution of the adjuvant, wherein the adjuvant which consists of one or a mixture of C₁₂-C₁₈ saturated fatty acid or their salts is colloidally dispersed in the solution; and
b) spray drying the clear solution of step a) using an inlet temperature between 120-135 °C, feed rate between 3.0 and 6.0 ml/min, air flow rate 600 L/h, nozzle between 0.5 and 1 mm, and aspirator 100%, wherein
the temperature of the clear solution in step a) and step b) is higher than or equal to 28°C to colloidally disperse the adjuvant in the solution, and wherein
said drug powder is an aminoglycoside antibiotic comprising particles of antibiotic which have a solubility in water of at least 3.0% w/v.

2. The drug powder according to claim 1, wherein the aminoglycoside antibiotic is selected from the group consisting of amikacin, gentamicin, kanamycin, neomycin, netilmicin, paromomycin, streptomycin and tobramycin.

3. The drug powder according to claim 2, wherein the aminoglycoside antibiotic is tobramycin or amikacin.

4. The drug powder according to anyone of claims 1-3, wherein the adjuvant is selected from the group consisting of lauric acid, myristic acid, palmitic acid, stearic acid, their mixtures and their salts.

5. The drug powder according to claim 4, wherein the adjuvant is stearic acid or a salt thereof.

6. The drug powder according to anyone of claims 1-5, wherein the drug particle is at least partially coated with an adjuvant in the amount from 0.25 to 2.0 w/w with respect to the weight of the drug.

7. The drug powder according to claim 6, wherein the drug particle is at least partially coated with an adjuvant in the amount from 0.5 to 1.5 w/w with respect to the weight of the drug.

8. The drug powder according to any one of claims 1-7, wherein the temperature of the clear solution in step a) and step b) is higher than or equal to 30 °C.

9. A process for the preparation of the drug powder according to anyone of claims 1-5 comprises the following steps;
a) preparing a clear solution of drug particles by mixing a water solution of the drug particles with an alcoholic solution of the adjuvant, wherein an adjuvant which consists of one or a mixture of C₁₂-C₁₈ saturated fatty acid or their salts is colloidally dispersed in the solution;
b) spray drying the clear solution of step a) using an inlet temperature between 120-135 °C, feed rate between 3.0 and 6.0 ml/min, air flow rate 600 L/h, nozzle between 0.5 and 1 mm, and aspirator 100%, wherein
the temperature of the clear solution in step a) and step b) is higher than or equal to 28°C to colloidally disperse the adjuvant in the solution, and wherein
said drug powder is an aminoglycoside antibiotic comprising particles of antibiotic which have a solubility in water of at least 3.0% w/v.

10. The process of claim 9 wherein the clear solution of drug particles is obtained by a mixture of a water solution of the drug with an alcoholic solution of the adjuvant.

11. The process according to claim 9 or 10, wherein alcohol used for the dissolution of the adjuvant is selected from the group consisting of ethanol, methanol, propanol or isopropanol.

12. The process according to anyone of claims 9 -11, wherein the solutions of the adjuvant and of drug are mixed in a ratio in the range from 2:8 to 5:5.

13. The process according to anyone of claims 9-12, wherein the temperature of the clear solution in step a) and step b) is higher than or equal to 30 °C.

14. A use of a drug powder of anyone of claims 1-8 for the manufacture of a medicament for the treatment of diseases derived from bacterial infections to be administered by inhalation.

15. The use of claim 14, wherein the disease is cystic fibrosis.

## Patentansprüche

1. Arzneimittelpulver zur Inhalation, welches Partikel umfasst, in denen der Arzneimittelgehalt gleich oder höher als 90% w/w ist, wobei die Arzneimittelpartikel zumindest teilweise mit einer Menge an Hilfsstoff überzogen sind, die bezogen auf das Gewicht des Arzneimittels niedriger als oder gleich 2,0% w/w ist, wobei der Hilfsstoff aus einer oder einem Gemisch von gesättigten C₁₂-C₁₈-Fettsäure(n) oder deren Salz(en) besteht, wobei das Arzneimittelpulver erhalten ist durch
a) Herstellen einer klaren Lösung der Arzneimittelpartikel durch Vermischen einer wässrigen Lösung der Arzneimittelpartikel mit einer alkoholischen Lösung des Hilfsstoffes, wobei der Hilfsstoff, der aus einer oder einem Gemisch von gesättigten C₁₂-C₁₈-Fettsäure(n) oder deren Salz(en) besteht, kolloidal in der Lösung dispergiert wird; und
b) Sprühtrocknen der klaren Lösung von Schritt a) unter Verwendung einer Einlasstemperatur zwischen 120 und 135 °C, einer Zuführungsrate zwischen 3,0 und 6,0 ml/min, einer Luftströmungsrate von 600 L/h, einer Düse zwischen 0,5 und 1 mm, und von 100% Aspirator, wobei
die Temperatur der klaren Lösung in Schritt a) und Schritt b) höher als oder gleich 28 °C ist, um den Hilfsstoff kolloidal in der Lösung zu dispergieren, und wobei
das Arzneimittelpulver ein Aminoglykosid-Antibiotikum ist, das Partikel aus Antibiotikum umfasst, die eine Löslichkeit in Wasser von mindestens 3,0% w/v aufweisen.

2. Arzneimittelpulver nach Anspruch 1, wobei das Aminoglykosid-Antibiotikum aus der Gruppe ausgewählt ist, die aus Amikacin, Gentamicin, Kanamycin, Neomycin, Netilmicin, Paromomycin, Streptomycin und Tobramycin besteht.

3. Arzneimittelpulver nach Anspruch 2, wobei das Aminoglykosid-Antibiotikum Tobramycin oder Amikacin ist.

4. Arzneimittelpulver nach einem der Ansprüche 1 bis 3, wobei der Hilfsstoff ausgewählt ist aus der Gruppe, die aus Laurinsäure, Myristinsäure, Palmitinsäure, Stearinsäure, deren Mischungen und deren Salzen besteht.

5. Arzneimittelpulver nach Anspruch 4, wobei der Hilfsstoff Stearinsäure oder ein Salz derselben ist.

6. Arzneimittelpulver nach einem der Ansprüche 1 bis 5, wobei das Arzneimittelpartikel zumindest teilweise mit einem Hilfsstoff in einer Menge von 0,25 bis 2,0 w/w bezogen auf das Gewicht des Arzneimittels überzogen ist.

7. Arzneimittelpulver nach Anspruch 6, wobei das Arzneimittelpartikel zumindest teilweise mit einem Hilfsstoff in einer Menge von 0,5 bis 1,5 w/w bezogen auf das Gewicht des Arzneimittels überzogen ist.

8. Arzneimittelpulver nach einem der Ansprüche 1 bis 7, wobei die Temperatur der klaren Lösung in Schritt a) und Schritt b) höher als oder gleich 30 °C ist.

9. Verfahren zur Herstellung des Arzneimittelpulvers nach einem der Ansprüche 1 bis 5, welches die folgenden Schritte umfasst:
a) Herstellen einer klaren Lösung von Arzneimittelpartikeln durch Vermischen einer wässrigen Lösung von Arzneimittelpartikeln mit einer alkoholischen Lösung des Hilfsstoffes, wobei ein Hilfsstoff, der aus einer oder einem Gemisch von gesättigten C₁₂-C₁₈-Fettsäure(n) oder deren Salz(en) besteht, kolloidal in der Lösung dispergiert wird;
b) Sprühtrocknen der klaren Lösung von Schritt a) unter Verwendung einer Einlasstemperatur zwischen 120 und 135 °C, einer Zuführungsrate zwischen 3,0 und 6,0 ml/min, einer Luftströmungsrate von 600 L/h, einer Düse zwischen 0,5 und 1 mm, und von 100% Aspirator, wobei
die Temperatur der klaren Lösung in Schritt a) und Schritt b) höher als oder gleich 28 °C ist, um den Hilfsstoff kolloidal in der Lösung zu dispergieren, und wobei
das Arzneimittelpulver ein Aminoglykosid-Antibiotikum ist, das Partikel aus Antibiotikum umfasst, die eine Löslichkeit in Wasser von mindestens 3,0% w/v aufweisen.

10. Verfahren nach Anspruch 9, wobei die klare Lösung von Arzneimittelpartikeln aus einer Mischung einer wässrigen Lösung des Arzneimittels mit einer alkoholischen Lösung des Hilfsstoffes erhalten wird.

11. Verfahren nach Anspruch 9 oder 10, wobei zum Auflösen des Hilfsstoffes verwendeter Alkohol ausgewählt ist aus der Gruppe bestehend aus Ethanol, Methanol, Propanol oder Isopropanol.

12. Verfahren nach einem der Ansprüche 9 bis 11, wobei die Lösungen des Hilfsstoffes und des Arzneimittels in einem Verhältnis im Bereich von 2 : 8 bis 5 : 5 vermischt werden.

13. Verfahren nach einem der Ansprüche 9 bis 12, wobei die Temperatur der klaren Lösung in Schritt a) und Schritt b) höher als oder gleich 30 °C ist.

14. Verwendung eines Arzneimittelpulvers nach einem der Ansprüche 1 bis 8 zur Herstellung eines Medikaments zur Behandlung von Krankheiten, die von bakteriellen Infektionen herrühren, für die Verabreichung mittels Inhalation.

15. Verwendung nach Anspruch 14, wobei die Krankheit zystische Fibrose ist.

## Revendications

1. Poudre médicamenteuse pour l'inhalation comprenant des particules dans lesquelles la teneur en médicament est égale ou supérieure à 90 % p/p, dans laquelle lesdites particules de médicament sont au moins partiellement revêtues d'une quantité d'un adjuvant inférieure ou égale à 2,0 % p/p par rapport au poids du médicament, ledit adjuvant étant constitué d'un seul ou d'un mélange d'acides gras saturés en C₁₂-C₁₈ ou de leurs sels, dans laquelle ladite poudre médicamenteuse est obtenue en
a) préparant une solution limpide des particules de médicament en mélangeant une solution aqueuse des particules de médicament avec une solution alcoolique de l'adjuvant, dans laquelle l'adjuvant, qui est constitué d'un seul ou d'un mélange d'acides gras saturés en C₁₂-C₁₈ ou de leurs sels, est dispersé de manière colloïdale dans la solution ; et en
b) séchant par pulvérisation la solution limpide de l'étape a) en utilisant une température d'entrée comprise entre 120 et 135 °C, un débit d'alimentation compris entre 3,0 et 6,0 ml/min, un débit d'air de 600 L/h, une buse comprise entre 0,5 et 1 mm, et un aspirateur à 100 %, dans laquelle
la température de la solution limpide à l'étape a) et à l'étape b) est supérieure ou égale à 28 °C pour disperser de manière colloïdale l'adjuvant dans la solution, et dans laquelle
ladite poudre médicamenteuse est un antibiotique aminoglycoside comprenant des particules d'antibiotique qui ont une solubilité dans l'eau d'au moins 3,0 % p/v.

2. Poudre médicamenteuse selon la revendication 1, dans laquelle l'antibiotique aminoglycoside est sélectionné parmi le groupe constitué de l'amikacine, la gentamicine, la kanamycine, la néomycine, la nétilmicine, la paromomycine, la streptomycine et la tobramycine.

3. Poudre médicamenteuse selon la revendication 2, dans laquelle l'antibiotique aminoglycoside est la tobramycine ou l'amikacine.

4. Poudre médicamenteuse selon l'une quelconque des revendications 1-3, dans laquelle l'adjuvant est sélectionné parmi le groupe constitué de l'acide laurique, l'acide myristique, l'acide palmitique, l'acide stéarique, leurs mélanges et leurs sels.

5. Poudre médicamenteuse selon la revendication 4, dans laquelle l'adjuvant est l'acide stéarique ou un sel de celui-ci.

6. Poudre médicamenteuse selon l'une quelconque des revendications 1-5, dans laquelle la particule de médicament est au moins partiellement revêtue d'un adjuvant en la quantité de 0,25 à 2,0 p/p par rapport au poids du médicament.

7. Poudre médicamenteuse selon la revendication 6, dans laquelle la particule de médicament est au moins partiellement revêtue d'un adjuvant en la quantité de 0,5 à 1,5 p/p par rapport au poids du médicament.

8. Poudre médicamenteuse selon l'une quelconque des revendications 1-7, dans laquelle la température de la solution limpide à l'étape a) et à l'étape b) est supérieure ou égale à 30 °C.

9. Procédé de préparation de la poudre médicamenteuse selon l'une quelconque des revendications 1-5 comprenant les étapes suivantes ;
a) préparer une solution limpide de particules de médicament en mélangeant une solution aqueuse des particules de médicament avec une solution alcoolique de l'adjuvant, dans lequel un adjuvant qui est constitué d'un seul ou d'un mélange d'acides gras saturés C₁₂-C₁₈ ou de leurs sels est dispersé de manière colloïdale dans la solution ;
b) sécher par pulvérisation la solution limpide de l'étape a) en utilisant une température d'entrée comprise entre 120 et 135 °C, un débit d'alimentation compris entre 3,0 et 6,0 ml/min, un débit d'air de 600 L/h, une buse comprise entre 0,5 et 1 mm, et un aspirateur à 100 %, dans lequel
la température de la solution limpide à l'étape a) et à l'étape b) est supérieure ou égale à 28 °C pour disperser de manière colloïdale l'adjuvant dans la solution, et dans lequel
ladite poudre médicamenteuse est un antibiotique aminoglycoside comprenant des particules d'antibiotique qui ont une solubilité dans l'eau d'au moins 3,0 % p/v.

10. Procédé de la revendication 9 dans lequel la solution limpide de particules de médicament est obtenue par un mélange d'une solution aqueuse du médicament avec une solution alcoolique de l'adjuvant.

11. Procédé selon la revendication 9 ou 10, dans lequel l'alcool utilisé pour la dissolution de l'adjuvant est sélectionné parmi le groupe constitué de l'éthanol, le méthanol, le propanol ou l'isopropanol.

12. Procédé selon l'une quelconque des revendications 9-11, dans lequel les solutions de l'adjuvant et du médicament sont mélangées dans un rapport dans la plage de 2:8 à 5:5.

13. Procédé selon l'une quelconque des revendications 9-12, dans lequel la température de la solution limpide à l'étape a) et à l'étape b) est supérieure ou égale à 30 °C.

14. Utilisation d'une poudre médicamenteuse de l'une quelconque des revendications 1-8 pour la fabrication d'un médicament pour le traitement de maladies dérivées d'infections bactériennes à administrer par inhalation.

15. Utilisation de la revendication 14, dans laquelle la maladie est la fibrose kystique.
